# EUROPEAN PATENT SPECIFICATION

(11) **EP 1 586 625 B1**
(45) Date of publication and mention of the grant of the patent: **03.06.2009**
(21) Application number: 03768383.6
(22) Date of filing: 26.12.2003
(51) Int. Cl.: C11D 1/10, A61K 8/44, A61Q 5/02, A61Q 19/10

(54) **NOVEL SURFACTANTS AND USE THEREOF**
NEUARTIGE TENSIDE UND IHRE VERWENDUNG
NOUVEAUX TENSIOACTIFS ET LEUR UTILISATION

(30) Priority: 07.01.2003 JP 2003000889
(43) Date of publication of application: 19.10.2005
(73) Proprietor: P & PF Co., Ltd., Ibaraki-shi, Osaka 567-0023 (JP)
(72) Inventor: SAITO, Yoshinobu, Ibaraki-shi, Osaka 567-0007 (JP); MATSUMOTO, Satoshi, Yokohama-shi, Kanagawa 223-0065 (JP); NAGAHAMA, Daiji, Takatsuki-shi, Osaka 569-0015 (JP); OKUDA, Takahiro, Ibaraki-shi, Osaka 567-0861 (JP); NISHINA, Tetsuo, Takatsuki-shi, Osaka 569-1022 (JP)
(74) Representative: Harrison, Robert John
(86) International application number: PCT/JP2003/017078
(87) International publication number: WO 2004/061060

(56) References cited:
- EP-A- 0 648 833
- JP-A- 7 331 281
- JP-A- 9 157 140
- JP-A- 11 180 836
- JP-A- 11 180 855
- JP-A- 11 323 378
- JP-A- 11 323 379
- JP-A- 11 323 380
- JP-A- 50 022 809
- JP-A- 53 126 006
- JP-A- 2000 345 190
- JP-A- 2001 040 390
- JP-A- 2002 020 267
- US-A- 4 273 684
- DATABASE WPI Section Ch, Week 198305 Derwent Publications Ltd., London, GB; Class D21, AN 1983-10624K XP002375985 & JP 57 205498 A (LION CORP) 16 December 1982 (1982-12-16)

## Description

This invention relates to a novel Surfactant and its use, more particularly to a detergent composition and an emulsion composition using the novel Surfactant.

Traditionally, soap is often used in cleansing agents such as face washes, body Shampoos and hair Shampoos in order to improve lathering ability and foam quality. As such, most popular soap is alkali metal salt soap (e.g. a sodium salt or potassium salt of a fatty acid), with such advantages as low cost, good lathering ability and creamy foam quality. On the other hand, however, the alkali metal soap has some defects. As a hair Shampoo, it makes the hair stiff and, as a face wash, it leaves the skin taut after washing.

To cope with these problems, Japanese Patent Application Laid-open No. 9-1 157688 proposes a detergent composition which contains, as an essential component, a fatty acid salt of either alkali metal N-methyltaurate or organic base N-methyltaurate.

Nevertheless, this detergent composition still causes stiff hair and taut skin after use.

Documents JP-11 323380, JP-A-11 323379 and JP-A-11 323379 disclose detergent compositions comprising N-acylamino acid salts (JP-11 323380: acylamino acid salts, JP-A-11 323379: anionic acylamino acid salt, JP-A-11 323379: acyl neutral amino acid salt and acyl acidic amino acid salt) and acidic amino acids or salts thereof. However, these detergent compositions simply contain N-acylamino acids salts and acidic amino acids or salts thereof.

Document JP-A-7 331281 discloses a detergent composition comprising (A) an N-acylglycine or its salt and (B) an amino acid. With respect to N-acylglycine, N-acylglycine is neutralized with metal salts (such as sodium salt) and basic amino acid salts.

Document JP-A-53-1 26006 discloses a detergent composition comprising an N-acyl acidic amino acid salt and an amino acid or salt thereof. With respect to the N-acyl aminoacid salt, an N-acyl acidic amino acid is neutralized with metal salts (such as sodium salt) or basic amino acid salts.

Document JP-A-50-022809 discloses a detergent composition comprising a long-chain N-acyl acidic amino acid salt, an aliphatic non-ionic surfactant and a basic amino acid salt (as an organic carboxylate). However, this detergent composition simply contains a long-chain N-acyl acidic aminoacid salt and a basic amino acid salt.

Document JP-A-2000 345190 discloses various amines as an N-acylamino acid and its counteraction. The counteraction to an N-acylamino acid represents various amines.

Document JP-A-2002-020267 discloses a skin cleansing agent containing acylglycine alkali metal salt, acylglutamine alkali salt, an organic acid and water.

Document JP-A-11 180855 discloses a skin washing agent composition containing an N-acylamino acid-type surfactant.

Document JP-A-9-157140 discloses a composition containing (A) at least one kind of component selected from a long-chain N-acylthreonine salt and a long-chain N-acylalanine salt (B) an aliphatic alka-nolamide, and (C) a betaine. As the component (A), this document discloses basic amino acid salts of a long-chain N-acylthreonine and a long-chain N-acylalanine.

Document JP-A-11-180836 discloses a composition containing (A) a basic amino acid derivative or salt thereof shown by Formula I and (B) a neutral amino acid or salt thereof and/or a low molecular-weight betaine. The component (A) is obtained by causing glycidyl ethers and/or epoxyalkanes (both being epoxy compounds) to react with basic amino acids.

Document JP-A-2001 -040390 discloses a detergent composition comprising a fatty acid and an alkali salt of an amino acid, wherein the equivalent ratio of the alkali salt of an amino acid is 1/5-5/1 relative to the carboxyl group in the fatty acid. As the fatty acid, this disclosure mentions caprylic acid, capric acid, lauric acid, myristic acid, palmitic acid, stearic acid, isostearic acid, oleic acid, coconut fatty acid, palm kernel fatty acid, and tallow fatty acid.

Document US-A-4273684 discloses a salt of a N-acyl acidic amino acid and a basic amino acid obtained by blending and neutralizing an N-acyl acidic amino acid and a basic amino acid. In this respect, the present invention employs an alkali salt of an amino acid whose alkali component is a weakly basic alkali salt of an amino acid having an ion pair which is obtained by blending and neutralizing a basic amino acid.

Document EP-A 648833 discloses a salt of an N-acylamino acid and a salt of a higher fatty acid.

Document JP-A-57 205498 discloses a salt of an N-acylamino acid and a salt of an N-acyl glutamate. This invention is made in order to solve such problems especially mentioned in regards to above JP-A-9-1 57688.

An object of the invention is to provide a detergent composition which ensures good lathering ability and foam quality and which prevents stiff hair or taut skin after use.

As a result of intensive researches for solving the problems, the inventors obtained a detergent composition whose cleansing component was a novel Surfactant obtained by specific blending an N-Cs-24 acylamino acid and a particular base (i.e. an alkali salt of an amino acid). They found that this detergent composition could ensure good lathering ability and foam quality and could prevent stiff hair or taut skin after use. They also found that an emulsion composition whose emulsifying component was the novel Surfactant could realize a good emulsion State. Based on these findings, the inventors accomplished the invention.

Namely, this invention concerns a Surfactant which is obtained by blending an N-C₈-24 acylamino acid and a defined salt of an amino acid, according to claim 1.

The invention comprises the features of claim 1.

The invention provides a surfactant characterised in comprising a blend of an acidic or neutral N-C₈-₂₄ acylamino acid and a base , wherein said base has an ion pair formed by an anion deriving from a carboxyl group of an amino acid and a cation deriving from alkali metals, alkaline earth metals, organic amines, basic amino acids, N-methyltaurine sodium, N-methyltaurine potassium, taurine sodium, taurine potassium, and said acidic or neutral N-C₈₋₂₄ acylamino acid and said base are blended and neutralised to form an ion pair having a pH of 5 to 9, where the amount of said base is 1.0 to 1.6 equivalents relative to 1 equivalent of N-C₈₋₂₄ acylamino acid and, in the case where the N-C₈₋₂₄ acylamino acid has two carboxl groups, is 1.3 to 2.3 equivalents relative to 2 equivalents of N-C₈₋₂₄ acylamino acid.

In a suitable embodiment, an amino acid in the alkali salt of an amino acid is preferably at least one member selected from the group consisting of acidic amino acids and neutral amino acids, and is preferably at least one member selected from α-amino acids. More preferably, an amino acid in the alkali salt of an amino acid is at least one member selected from the group consisting of glycine, trimethylglycine, alanine, serine, proline, hydroxyproline, glutamine, glutamic acid, asparagine, aspartic acid, and glycylglycine. A particularly preferable amino acid is at least one member selected from the group consisting of glycine, trimethylglycine, alanine, serine, glutamic acid, and glycylglycine.

In a suitable embodiment, an alkali in the alkali salt of an amino acid is preferably at least one member selected from the group consisting of sodium, potassium, triethanolamine, and N-methyltaurine sodium.

In a suitable embodiment, a C₈₋₂₄ acyl in the N-C₈₋₂₄ acylamino acid is preferably a C₁₂₋₁₈ acyl, and more preferably at least one member selected from the group consisting of lauroyl, myristoyl, palmitoyl, stearoyl, isostearoyl, oleoyl, coconut oil acyl, palm oil acyl, palm kernel oil acyl, tallow acyl, and hydrogenated tallow acyl.

In a suitable embodiment, an amino acid in the N-C₈₋₂₄ acylamino acid is preferably at least one member selected from the group consisting of acidic amino acids and neutral amino acids, and more preferably at least one member selected from the group consisting of glutamic acid, aspartic acid, sarcosine, alanine, glycine, β-alanine, N-methyl-β-alanine, and glutamine.

The invention also concerns a detergent composition which comprises the above surfactant as a cleansing component. Preferably, the surfactant content is 3 to 50% by weight.

The invention further concerns an emulsion composition which comprises the above surfactant as an emulsifying component. Preferably, the surfactant content is 0.1 to 5% by weight.

### Details:

### < N-C₈₋₂₄ acylamino acid>

In this invention, the "C₈₋₂₄ acyl" in the "N-C₈₋₂₄ acylamino acid" is an acyl having 8 to 24 carbon atoms, which may be linear or branched and may be saturated or unsaturated (i.e. an acyl deriving from a fatty acid having 8 to 24 carbon atoms). The number of carbon atoms is preferably 12 to 18. Specific suitable examples of the "C₈₋₂₄ acyl" include acyls deriving from saturated fatty acids such as lauroyl, myristoyl, palmitoyl, stearoyl and isostearoyl; acyls deriving from unsaturated fatty acids such as oleoyl; mixtures thereof such as coconut oil acyl, palm oil acyl, palm kernel oil acyl, tallow acyl, hydrogenated tallow acyl.

In this invention, the "amino acid" in the "N-C₈₋₂₄ acylamino acid" includes not only α-amino acids but also β-amino acids, γ-amino acids, δ-amino acids, N-alkyl-substituted (preferably C₁₋₄) amino acids, and the like. An "amino acid" is acidic or neutral and may specifically be glutamic acid, aspartic acid, sarcosine, alanine, glycine, β-alanine, N-methyl-β-alanine, glutamine. The "amino acid" as called herein encompasses any stereoisomer of amino acids (e.g. For α-amino acids, all of d-, 1- and dl-stereoisomers fall into the "amino acid".).

Specific suitable examples of the "N-C₈₋₂₄ acylamino acid" are N-lauroyl glutamic acid, N-myristoyl glutamic acid, N-palmitoyl glutamic acid, N-stearoyl glutamic acid, N-isostearoyl glutamic acid, N-oleoyl glutamic acid, N-coconut oil acyl glutamic acid, N-palm oil acyl glutamic acid, N-palm kernel oil acyl glutamic acid, N-tallow acyl glutamic acid, N-hydrogenated tallow acyl glutamic acid;

N-lauroyl aspartic acid, N-myristoyl aspartic acid, N-palmitoyl aspartic acid, N-stearoyl aspartic acid, N-isostearoyl aspartic acid, N-oleoyl aspartic acid, N-coconut oil acyl aspartic acid, N-palm oil acyl aspartic acid, N-palm kernel oil acyl aspartic acid, N-tallow acyl aspartic acid, N-hydrogenated tallow acyl aspartic acid;

N-lauroyl sarcosine, N-myristoyl sarcosine, N-palmitoyl sarcosine, N-stearoyl sarcosine, N-isostearoyl sarcosine, N-oleoyl sarcosine, N-coconut oil acyl sarcosine, N-palm oil acyl sarcosine, N-palm kernel oil acyl sarcosine, N-tallow acyl sarcosine, N-hydrogenated tallow acyl sarcosine;

N-lauroyl alanine, N-myristoyl alanine, N-palmitoyl alanine, N-stearoyl alanine, N-isostearoyl alanine, N-oleoyl alanine, N-coconut oil acyl alanine, N-palm oil acyl alanine, N-palm kernel oil acyl alanine, N-tallow acyl alanine, N-hydrogenated tallow acyl alanine;

N-lauroyl glycine, N-myristoyl glycine, N-palmitoyl glycine, N-stearoyl glycine, N-isostearoyl glycine, N-oleoyl glycine, N-coconut oil acyl glycine, N-palm oil acyl glycine, N-palm kernel oil acyl glycine, N-tallow acyl glycine, N-hydrogenated tallow acyl glycine;

N-lauroyl-β-alanine, N-myristoyl-β-alanine, N-palmitoyl-β-alanine, N-stearoyl-β-alanine, N-isostearoyl-β-alanine, N-oleoyl-β-alanine, N-coconut oil acyl-β-alanine, N-palm oil acyl-β-alanine, N-palm kernel oil acyl-β-alanine, N-tallow acyl-β-alanine, N-hydrogenated tallow acyl-β-alanine;

N-lauroyl-N-methyl-β-alanine, N-myristoyl-N-methyl-β-alanine, N-palmitoyl-N-methyl-β-alanine, N-stearoyl-N-methyl-β-alanine, N-isostearoyl-N-methyl-β-alanine, N-oleoyl-N-methyl-β-alanine, N-coconut oil acyl-N-methyl-β-alanine, N-palm oil acyl-N-methyl-β-alanine, N-palm kernel oil acyl-N-methyl-β-alanine; N-tallow acyl-N-methyl-β-alanine, N-hydrogenated tallow acyl-N-methyl-β-alanine;

N-lauroyl glutamine, N-myristoyl glutamine, N-palmitoyl glutamine, N-stearoyl glutamine, N-isostearoyl glutamine, N-oleoylglutamine, N-coconut oil acyl glutamine, N-palm oil acyl glutamine, N-palm kernel oil acyl glutamine, N-tallow acyl glutamine, and N-hydrogenated tallow acyl glutamine.

Among them, particularly suitable N-C₈₋₂₄ acylamino acids are N-coconut oil acyl glutamic acid, N-coconut oil acyl aspartic acid, N-coconut oil acyl glycine, N-coconut oil acyl-N-methyl-β-alanine, and N-coconut oil acyl glutamine.

### <Alkali salt of amino acid>

In the "alkali salt of an amino acid" as called in the description, an ion pair is formed by an anion deriving from a carboxyl group of an amino acid and a cation as defined in claim 1.
In this invention, the "amino acid" in the "alkali salt of an amino acid" includes not only α-amino acids but also β-amino acids, γ-amino acids, δ-amino acids, N-alkyl-substituted (preferably C₁₋₄) amino acids, and also includes oligopeptides composed of two to five such amino acids. Specific examples are glycine, sarcosine, trimethylglycine, alanine, β-alanine, valine, leucine, isoleucine, serine, threonine, aspartic acid, glutamic acid, asparagine, glutamine, cysteine, cystine, methionine, phenylalanine, tyrosine, proline, hydroxyproline, glycylglycine, glycyl glycylglycine, glycylproline. The "amino acid" as called herein encompasses any stereoisomer of amino acids (e.g. For α-amino acids, all of d-, 1- and dl-stereoisomers fall into the "amino acid".).

In this invention, the "amino acid" is acidic or neutral, and is preferably an α-amino acid. Specific suitable examples of the "amino acid" are glycine, trimethylglycine, alanine, serine, proline, hydroxyproline, glutamine, glutamic acid, asparagine, aspartic acid, and glycylglycine. Among them, particularly suitable amino acids are glycine, trimethylglycine, alanine, serine, glutamic acid, and glycylglycine. When the surfactant according to the invention is used as a detergent component, these amino acids contribute to remarkable lathering ability.

In this invention, the "alkali" in the "alkali salt of an amino acid" includes alkali metals such as sodium, potassium and lithium; alkaline earth metals such as calcium and magnesium; organic amines such as ethanolamines (including monoethanolamine, diethanolamine,and triethanolamine),basicamino acids (e.g. lysine, arginine, histidine), N-methyltaurine sodium, N-methyltaurine potassium, taurine sodium, taurine potassium. Specific suitable examples of the "alkali" are sodium, potassium, triethanolamine, and N-methyltaurine sodium.

In the case where an amino acid has two carboxyl groups (e.g. glutamic acid, aspartic acid), the preferable amount of alkali is 1.2 to 2 equivalents, particularly 1.5 to 2 equivalents, relative to 2 equivalents of amino acid (Namely, the preferable amount of alkali is 1.2 to 2 moles, particularly 1.5 to 2 moles, relative to 1 mole of amino acid.).

Specific suitable examples of the "alkali salt of an amino acid" in the invention are sodium glycinate, sodium salt of trimethylglycine, sodium alanate, sodium salt of serine, sodium prolinate, sodium hydroxyprolinate, sodium salt of glutamine, sodium glutamate (sodium: 1.2 to 2 equivalents), sodium salt of asparagine, sodium aspartate (sodium: 1.2 to 2 equivalents), sodium glycylglycinate, potassium glycinate, triethanolamine glycinate, N-methyltaurine sodium salt of glycine, potassium glutamate (potassium: 1.2 to 2 equivalents), triethanolamine glutamate (triethanolamine: 1.2 to 2 equivalents), N-methyltaurine sodium glutamate (N-methyltaurine sodium: 1.2 to 2 equivalents), potassium glycylglycinate, etc. Among them, particularly suitable alkali salts of amino acids are sodium glutamate (sodium: 1.2 to 2 equivalents), N-methyltaurine sodium glutamate (N-methyltaurine sodium: 1.2 to 2 equivalents), sodium glycinate, sodium alanate, sodium salt of serine, sodium salt of trimethylglycine, and sodium glycylglycinate.

### <Surfactant>

The amount of alkali salt of an amino acid is 1.0 to 1.6 equivalents, particularly 1.0 to 1.4 equivalents, relative to 1 equivalent of N-C₈₋₂₄ acylamino acid. However, where the N-C₈₋₂₄ acylamino acid has two carboxyl groups (e.g. an N-C₈₋₂₄ acylglutamic acid, an N-C₈₋₂₄ acylaspartic acid), the preferable amount of alkali salt of an amino acid is 1.3 to 2.3 equivalents, particularly 1.4 to 2.0 equivalents, relative to 2 equivalents of N-C₈₋₂₄ acylamino acid.

The surfactant according to the invention is obtained by blending an N-C₈₋₂₄ acylamino acid and an alkali salt of an amino acid. To give a specific suitable example, the specific suitable examples of the "N-C₈₋₂₄ acylamino acid" and the "alkali salt of amino acid", as mentioned earlier, may be blended in various combinations and neutralized. Above all, the surfactants obtained by the following combinations are particularly suitable.

A blend of N-coconut oil acyl glutamic acid and sodium glutamate (sodium glutamate: 1.3 to 2.3 equivalents, Na content in sodium glutamate: 1.2 to 2 equivalents);

A blend of N-coconut oil acyl glutamic acid and sodium salt of serine (sodium salt of serine: 1.3 to 2.3 equivalents);

A blend of N-coconut oil acyl glutamic acid and sodium alanate (sodium alanate: 1.3 to 2.3 equivalents);

A blend of N-coconut oil acyl aspartic acid and sodium glutamate (sodium glutamate: 1.3 to 2.3 equivalents, Na content in sodium glutamate: 1.2 to 2 equivalents);

A blend of N-coconut oil acyl glycine and sodium glycinate (sodium glycinate: 1.0 to 1.6 equivalents); and

A blend of N-coconut oil acyl-N-methyl-β-alanine and sodium glutamate (sodium glutamate: 1.0 to 1.6 equivalents, Na content in sodium glutamate: 1.2 to 2 equivalents).

To prepare the surfactant according to the invention, an aqueous solution containing an alkali salt of an amino acid may be added with stirring to an N-C₈₋₂₄ acylamino acid, or an N-C₈₋₂₄ acylamino acid may be added with stirring to an aqueous solution containing an alkali salt of an amino acid. The resulting surfactant, obtained in the form of an aqueous solution, may be used as it is, for example, as a cleansing component or an emulsifying component.

The N-C₈₋₂₄ acylamino acid is weakly acidic, whereas the alkali salt of an amino acid is weakly basic. Hence, for the purpose of neutralization, it is advisable that the alkali salt of an amino acid is used in slight excess of its theoretical amount. Although its amount may vary with the types of N-C₈₋₂₄ acylamino acid and alkali salt of an amino acid to be used, the preferable amount of alkali salt of an amino acid is 1.0 to 1.6 moles, particularly 1.0 to 1.4 moles, relative to 1 mole of N-C₈₋₂₄ acylamino acid. In the case of an N-C₈₋₂₄ acylglutamic acid or an N-C₈₋₂₄ acylaspartic acid, the amount of alkali salt of an amino acid is preferably 1.3 to 2.3 moles, particularly 1.4 to 2.0 moles, relative to 1 mole of N-C₈₋₂₄ acylamino acid. Having said that, it is not desirable to employ an alkali salt of an amino acid in an overly excessive amount because the resulting surfactant has such a high pH as to deteriorate lathering ability when used as a cleansing component. For preparation of the surfactant, a temperature from 50 to 80°C is suitable.

The thus prepared surfactant of the invention has a pH of from 5 to 9, preferably from 6 to 8, and ensures good lathering ability.

The detergent composition according to the invention contains this surfactant (preferably, in the form of an aqueous solution) as a cleansing component. As already mentioned, the surfactant is obtained by blending an N-C₈₋₂₄ acylamino acid and an alkali salt of an amino acid, and may be used singly or in combination.

In this invention, the amount of surfactant may vary with the type of surfactant but is preferably from 3 to 50% by weight of the composition. Acomposition with a surfactant content of less than 3% by weight does not lather well, and a composition with a surfactant content of more than 50% by weight does not dissolve well, thus neither being desirable. Additionally, the suitable amount also changes with the form of the detergent composition. For example, a more preferable content of the surfactant is 3 to 40% by weight for a liquid composition and 10 to 50% by weight for a solid composition.

Where necessary, in addition to the essential surfactant, the detergent composition of the invention may contain additives which are usually blended into a detergent composition. The additives may be, for example, anionic surfactants such as fatty acid salts (soap), N-long chain acylamino acid salts, alkyl sulfate esters, polyoxyethylene alkyl ether sulfates, and hydroxyalkyl ether carboxylates; amphoteric surfactants such as imidazoline-type amphoteric surfactants and betaine-type amphoteric surfactants; nonionic surfactants such as polyoxyethylene alkyl ethers, polyoxyethylene fatty acid esters, sucrose fatty acid esters, alkylglucosides, and multitol hydroxy aliphatic ethers; cationic surfactants such as trimethylalkyl ammonium chlorides; humectants such as glycerine, diglycerine, 1,3-butylene glycol, propylene glycol, dipropylene glycol, polyethylene glycol, saccharose, sorbitol, and sodium hyaluronate; chelating agents such as edetates; plant extracts such as Swertia japonica, Paeonia lactiflora, Iris, Equisetum arvense (horsetail), aloe, Matricaria chamomilla (chamomile), eucalyptus oil, and dipotassium glycyrrhizinate; drugs such as tranexamic acid and arbutin; fragrances; colorants; preservatives.

Containing the above-mentioned surfactant as a cleansing component, the detergent composition of the invention does not make the hair stiff when used for a hair shampoo, and does not leave the skin taut after washing when used for a face wash. Further, in terms of lathering ability and foam quality, this detergent composition is superior to traditional components like sodium salts of fatty acids and sodium salts of N-acylamino acids.

The detergent composition of the invention can be used in any known forms, e.g. in solid, liquid, cream, foam, or powder form. Besides, the detergent composition can suit any known applications including hair shampoos and body shampoos; face washes; dish detergents and laundry detergents.

The detergent composition of the invention can be prepared in a known manner. By way of example, the detergent composition of the invention is provided as a liquid detergent by a common production process. To be specific, the surfactant is prepared in advance by blending an N-C₈₋₂₄ acylamino acid and an aqueous solution containing an alkali salt of an amino acid. A mixture of this surfactant, other ingredients and a solvent (e.g. water, alcohol) is dissolved under heating around 50 to 70°C to give a liquid detergent. Incidentally, the surfactant may be prepared not prior to but during this production process, by blending an N-C₈₋₂₄ acylamino acid and an aqueous solution containing an alkali salt of an amino acid.

Further, the detergent composition of the invention is provided as a solid detergent by a common production process. To be specific, the surfactant is prepared in advance by blending an N-C₈₋₂₄ acylamino acid and an aqueous solution containing an alkali salt of an amino acid. This surfactant is mixed with other ingredients at around 60 to 80°C, and the mixture is made into neat soap by a common framing method. The neat soap is subjected to molding, aging (where necessary) and shaping processes in a normal manner, to give a solid detergent. Incidentally, the surfactant may be prepared not prior to but during the neat soap making process, by blending an N-C₈₋₂₄ acylamino acid and an aqueous solution containing an alkali salt of an amino acid.

The emulsion composition according to the invention contains the above-mentioned surfactant (preferably, in the form of an aqueous solution) as an emulsifying component. As already mentioned, the surfactant is obtained by blending an N-C₈₋₂₄ acylamino acid and an alkali salt of an amino acid, and may be used singly or in combination.

In this invention, the amount of surfactant may vary with the type of surfactant but is preferably from 0.1 to 5% by weight of the composition. If the surfactant content is too much or too little, the resulting composition may not be emulsified sufficiently and thus is undesirable.

The emulsion composition of the invention contains the essential surfactant, water, and oil components usually blended into an emulsion composition (liquid oils, solid oils, waxes, hydrocarbon oils, higher fatty acids, higher alcohols, synthetic ester oils, silicone oils, etc.). Where necessary, the emulsion composition may additionally contain other surfactants which are usually blended into an emulsion composition (anionic surfactants, amphoteric surfactants, nonionic surfactants (lipophilic, hydrophilic), and cationicsurfactants), and additives. The additives may be, for example, humectants, powdery components, water-soluble polymers, viscosity improvers, UV absorbents, metal ion sequestering agents, lower alcohols, polyhydric alcohols, saccharides (monosaccharides, oligosaccharides, and polysaccharidss), amino acids, organic amines, pH controlling agents, antioxidants, auxiliary antioxidants, preservatives, antiphlogistics, whitening agents, various extracts, activating agents, circulation stimulants, anti-seborrhea agents, anti-inflammatory agents, etc.

Containing the above-mentioned surfactant as an emulsifying component, the emulsion composition of the invention ensures a good emulsion state.

The emulsion composition of the invention can be used in any known forms such as cream, liquid or gel. Besides, the emulsion composition can suit any known applications including cosmetics (creams, milky lotions, and serums), pharmaceuticals, medicated cosmetics, and foods, to give a few examples.

The emulsion composition of the invention can be prepared in a known manner. By way of example, the emulsion composition of the invention is provided as an emulsion cream by a common production process. To be specific, the surfactant is prepared in advance by blending an N-C₈₋₂₄ acylamino acid and an aqueous solution containing an alkali salt of an amino acid. Under heating around 50 to 70°C, this surfactant, oil components, aqueous components, additives and others are mixed and emulsified (with use of a homomixer or the like) to give an emulsion cream composition Incidentally, the surfactant may be prepared not prior to but during this production process, by blending an N-C₈₋₂₄ acylamino acid and an aqueous solution containing an alkali salt of an amino acid.

The surfactant of the invention can be used not only for a cleansing agent and an emulsifier, but also for various known surfactant applications, examples of which may be a scouring agent, a foaming agent, a defoaming agent, a demulsifier, a dispersant, a wetting agent, a dissolving agent, a lustering agent, a delustering agent, a lubricating agent, an anti-slipping agent, a softening agent, a dye fixing agent, a dye leveling agent, a dye retarding agent, a dye discharging agent, a water repellent, a flame repellent, an antistatic agent, a flotation agent, an antirust, an anticorrosive, a disinfectant, etc.

### EXAMPLES

The invention is described in greater detail by the following non-limitative examples.

### Preparation of aqueous solutions containing alkali salts of amino acids

Amino acids, an alkaline aqueous solution and ion exchange water were employed in predetermined amounts as shown in Table 1. Aqueous solutions containing alkali salts of amino acids (Samples 1 to 7) were prepared by adding the respective amino acids to the ion exchange water, followed by addition of the alkaline aqueous solution with stirring at 70°C.

**Table 1**

| | Aqueous solution containing alkali salt of amino acid | Amino acid | | Alkaline aqueous solution NaOH(50%) | Ion exchange water |
|---|---|---|---|---|---|
| Sample 1 | sodium glycinate <20%t | glycine | 15.17g | 16.48g | 68.05g |
| Sample 2 | sodium glutamate (20%) (sodium: 1.8 eq) | glutamic acid | 15.76g | 15.42g | 68.82g |
| Sample 3 | sodium alanate (20%) | alanine | 16.04g | 14.4g | 69.56g |
| Sample 4 | sodium salt of serine (20%) | serine | 16.54g | 12.59g | 70,87g |
| Sample 5 | sodium prolinate (20%) | proline | 16.79g | 11.67g | 71.54g |
| Sample 6 | sodium hydroxyprolinate (20%) | hydroxyproline | 17.13g | 10.45g | 72.42g |
| Sample 7 | sodium salt of trimethylglycine (20%) | trimethylglycine | 16.72g | 11.42g | 71.86g |

### Examples 1 to 7

N-coconut oil acyl glutamic acid, aqueous solutions containing alkali salts of amino acids, and ion exchange water were used in predetermined amounts as shown in Table 2. Surfactants of Examples 1 to 7 were prepared by adding N-coconut oil acyl glutamic acid to the ion exchange water, followed by addition of, with stirring at 60°C, the respective aqueous solutions containing alkali salts of amino acids. In Tables 2 and 3, these surfactants (30% aqueous solutions) were indicated as "N-coconut oil acyl glutamic acid-alkali salt of amino acid".

**Table 2**

| | Surfactant (30% aqueous solution)^{*1} | N-coconut oil acyl glutamic acid | Aqueous solution containing alkali salt of amino acid | Ion exchange Water |
|---|---|---|---|---|
| Example 1 | N-coconut oil acyl glutamic acid-sodium glycinate | 20.52g | Sample 1, 47.4g | 32.08g |
| Example 2 | N-coconut oil acyl glutamic acid-sodium glutamate | 15.72g | Sample 2, 71.4g | 12.88g |
| Example 3 | N-coconut oil acyl glutamic acid-sodium alanate | 19.62g | Sample 3, 51.84g | 28.55g |
| Example 4 | N-coconut oil acyl glutamic acid-sodium salt of serine | 18.72g | Sample 4, 56.4g | 24.88g |
| Example 5 | N-coconut oil acyl glutamic acid -sodium prolinate | 18.18g | Sample 5, 59.16g | 22.66g |
| Example 6 | N-coconut oil acyl glutamic acid -sodium hydroxyprolinate | 17.34g | Sample 6, 63.3g | 19.66g |
| Example 7 | N-coconut oil acyl glutamic acid -sodium salt of trimethylglycine | 17.91g | Sample 7, 60.45g | 42.54g |

| | | | | |
|---|---|---|---|---|
| ^{*1} N-coconut oil acyl glutamic acid-alkali salt of amino acid (alkali salt of amino acid: 1.6 eq) | | | | |

The surfactants prepared in Examples 1 to 7 were evaluated for lathering ability, feel in use (refreshed feel and moistness), and stability. In Comparative Examples 1 and 2, sodium N-coconut oil acyl glutamate (30% aqueous solution) and potassium salt of N-coconut oil acylglycine (30% aqueous solution) were used as the surfactant and tested in the same manner, respectively. The results are shown in Table 3.

**Table 3**

| | Surfactant (30% solution) | Lathering ability | Feel in use | | | Stability |
|---|---|---|---|---|---|---|
| | | | Refreshed feel | Moistness | | |
| | | | | after 5 mins | after 12 hrs. | |
| Example 1 | N-coconut oil acyl glutamic acid -sodium glycinate | very good | good | good | good | very good |
| Example 2 | N-coconut oil acyl glutamic acid -sodium glutamate | good | good | very good | very good | very good |
| Example 3 | N-coconut oil acyl glutamic acid -sodium alanate | good | good | very good | very good | very good |
| Example 4 | N-coconut oil acyl glutamic acid -sodium acid of serine | very good | good | very good | very good | good |
| Example 5 | N-coconut oil acyl glutamic acid -sodium prolinate | good | good | very good | very good | good |
| Example 6 | N-coconut oil acyl glutamic acid -sodium hydroxyprolinate | good | good | very good | very good | good |
| Example 7 | N-coconut oil acyl glutamic acid -sodium salt of trimethylglycine | very good | good | very good | good | good |
| Comp.Ex. 1 | sodium N-coconut oil acyl glutamate | fair | good | fair | bad | good |
| Comp.Ex. 2 | potassium salt of N-coconut oil acylglycine | fair | good | fair | bad | good |

### <Methods of evaluation>

### 1. Lathering ability

Using artificial hard water containing 70 ppm of calcium carbonate, 1% aqueous solutions of the surfactant samples were prepared. By a mixer rotating at a given speed, each solution was stirred at 40°C for a predetermined time. Lathering ability was judged by measurement of the amount of generated lather, according to the following criteria.

| | |
|---|---|
| very good: | 2200 ml or more |
| good : | 2000 ml or more, but less than 2200ml |
| fair : | 1800 ml or more, but less than 2000ml |
| bad : | less than 1800 ml |

### 2. Feel in use (Refreshed feel and moistness)

Twenty female subjects (in their 20's and 30's) made the surfactant samples lather in the hands and washed their face as they usually did. After washing, the face conditions (refreshed feel, and moistness after 5 minutes and after 12 hours) were evaluated according to the following criteria.

### Refreshed feel

| | |
|---|---|
| very good: | excellently refreshed |
| good : | sufficiently refreshed |
| fair : | refreshed as usual |
| bad : | sticky |

### Moistness

| | |
|---|---|
| very good: | excellently moist |
| good : | sufficiently moist |
| fair : | moist as usual |
| bad : | taut |

### 3. Stability

The surfactant samples were stored at 37°C for four weeks, while another set of surfactant samples (as standard items) were stored at -5°C for four weeks. Five specialists compared these items and judged their stability, based on the degree of discoloration.

| | |
|---|---|
| very good: | No discoloration relative to the standard item |
| good | : Acceptable slight discoloration relative to the standard item |
| bad | : Unacceptable discoloration relative to the standard item |
| very bad: | Unacceptable severe discoloration relative to the standard item |

As shown in Table 3, surfactants of Examples 1 to 7 lathered well, gave refreshed feel after use, and ensured moistness 12 hours later. Further, they showed good stability.

In contrast, the surfactants of Comparative Examples 1 and 2 were somewhat inferior in lathering ability. Besides, they left taut feel after 12 hours of use.

The next Examples concern formulations using the surfactant of the invention. The surfactant obtained by blending an N-C₈₋₂₄ acylamino acid and an alkali salt of an amino acid is mentioned herein as "N-C₈₋₂₄ acylamino acid-alkali salt of amino acid".

### Example 8 (preparation of hair shampoo)

| | % by weight |
|---|---|
| Lauryl aminoacetic acid betaine (30% solution) | 10 |
| Sodium lauryl sulfate (30% solution) | 10 |
| Monoethanolamine laurate | 5 |
| Ethylene glycol fatty acid ester | 1.5 |
| Propylene glycol | 3 |
| Edetate | 0.1 |
| N-coconut oil acyl glutamic acid -sodium glycinate (30% solution) (sodium glycinate: 1.5 eq) | 20 |
| Pearl protein extracts | 0.1 |
| Purified water | rest |
| Total | 100 |

To obtain a hair shampoo, the above ingredients were evenly dissolved at 70°C and then, with stirring, cooled down to 35°C. The hair shampoo was mild to the hair and skin, lathered well, and left the hair silky after washing.

### Example 9 (preparation of body shampoo)

| | % by weight |
|---|---|
| N-lauroyl glycine potassium | 5 |
| Cocamidopropyl betaine (30% solution) | 15 |
| N-coconut oil acyl glutamic acid -sodium salt of serine (30% solution) (sodium salt of serine: 1.4 eq) | 5 |
| N-myristoyl glutamic acid -sodium glutamate (30% solution) (sodium glutamate: 1.6 eq) (Na content in sodium glutamate : 2.0 eq) | 3 |
| 1,3-Butylene glycol | 10 |
| Edetate | 0.1 |
| Diethanolamine laurate | 3 |
| Glycerine | 5 |
| Purified water | rest |
| Total | 100 |

To obtain a body shampoo, the above ingredients were evenly dissolved at 75°C and then, with stirring, cooled down to 35°C. The body shampoo lathered well, and gave refreshed feel and remarkable moistness after washing.

### Example 10 (preparation of liquid face wash)

| | % by weight |
|---|---|
| Lauryl imidazoliniumbetaine (30% solution) | 10 |
| N-lauroyl aspartic acid-sodium alanate (30% solution) (sodium alanate: 1.5 eq) | 3 |
| N-coconut oil acyl glutamic acid -sodium alanate (30% solution) (sodium alanate: 1.5 eq) | 15 |
| Ethylene glycol fatty acid ester | 2 |
| Propylene glycol | 10 |
| Edetate | 0.1 |
| Fragrances | 0.3 |
| Purified water | rest |
| Total | 100 |

To obtain a liquid face wash, the above ingredients were evenly dissolved at 70°C and then, with stirring, cooled down to 35°C. The liquid face wash lathered well, and left the skin moist after washing.

### Example 11 (preparation of face wash cream)

| | % by weight |
|---|---|
| Glycerine | 18 |
| Sorbitol solution (70%) | 5 |
| Polyethylene glycol 1500 | 9 |
| Potassium salt of N-coconut oil acyl glycine | 25 |
| N-coconut oil acyl glutamic acid | 12.81 |
| Sodium glycinate | 7.8 |
| Ion exchange water | 10 |
| Ethylene glycol distearate | 2 |
| Stearyl monoglyceride | 1 |
| Isostearyl monoglyceride | 1 |
| Sodium N-coconut oil acyl-N-methyltaurate | 5 |
| Polyethylene powders | 3 |
| Chamomile extract | 0.1 |
| Citric acid | 3 |
| Fragrances | 0.5 |
| Ion exchange water | rest |
| Total | 100 |

Glycerine, sorbitol solution, polyethylene glycol 1500, potassium salt of N-coconut oil acyl glycine, and N-coconut oil acyl glutamic acid were dissolved at 75°C. This mixture was neutralized with addition of a premixed solution in which sodium glycinate and ion exchange water (10% by weight) were evenly dissolved. Added to the neutralized mixture were ethylene glycol distearate, stearyl monoglyceride, isostearyl monoglyceride, sodium N-coconut oil acyl-N-methyltaurate, polyethylene powders, chamomile extract, citric acid, fragrances and the rest of the ion exchange water. As a result, a soap solution was obtained. With stirring, the soap solution was cooled down to 40°C to give a face wash cream. The face wash cream lathered well, and gave refreshed feel and moistness after washing. The face wash cream did not irritate the skin.

### Example 12 (preparation of face wash foam)

| | % by weight |
|---|---|
| Glycerine | 5 |
| Dipropylene glycol | 10 |
| 1,3-Butylene glycol | 3 |
| N-coconut oil acyl glutamic acid | 2.6 |
| Sodium myristate | 1 |
| Glutamic acid | 1.8 |
| Caustic soda (40%) | 2.5 |
| Ion exchange water | 5 |
| Lauryl aminoacetic acid betaine (30% solution) | 3.5 |
| Edetate | 0.2 |
| Eucalyptus oil | 0.05 |
| Ion exchange water | rest |
| Total | 100 |

Glycerine, dipropylene glycol, 1,3-butylene glycol, N-coconut oil acyl glutamic acid, and sodium myristate were dissolved at 75°C. This mixture was neutralized with addition of a premixed solution in which glutamic acid, caustic soda and ion exchange water (5% by weight) were evenly dissolved. Further added to the neutralized mixture were lauryl aminoacetic acid betaine, edetate, eucalyptus oil and the rest of the ion exchange water. As a result, a soap solution was obtained. With stirring, this soap solution was cooled down to 40°C to give a liquid face wash, which was charged into a certain pump foaming bottle. The resulting face wash foam lathered well, and gave refreshed feel and moistness after washing. The face wash foam did not irritate the skin.

### Example 13 (preparation of solid white soap)

| | % by weight |
|---|---|
| Sodium alkyl isethionate of palm fatty acid | 40 |
| N-coconut oil acyl glutamic acid -sodium glycinate | 10 |
| (sodium glycinate: 1.4 eq) | |
| Stearic acid | 30 |
| Behenyl alcohol | 10 |
| Ion exchange water | 10 |
| Total | 100 |

The above ingredients were dissolved and mixed at 80°C. The mixture was poured into a given mold and allowed to harden at a room temperature for five hours. The hardened product was removed from the mold to give solid white soap. The solid white soap lathered well, left the skin moist after washing, and was mild to the skin.

### Example 14 (preparation of milky lotion)

| | % by weight |
|---|---|
| Cetostearyl alcohol | 3 |
| Hydrogenated palm oil | 2 |
| Mineral oil | 4 |
| N-stearoyl glutamic acid-sodium glutamate (sodium glutamate: 1.7 eq) (Na content in sodium glutamate: 2.0 eq) | 2 |
| Sodium stearate | 1 |
| Methyl paraben | 0.1 |
| Glycerine | 5 |
| 1,3-Butylene glycol | 8 |
| Ion exchange water | rest |
| Total | 100 |

Ion exchange water, glycerine, 1,3-butylene glycol and methyl paraben were mixed and dissolved at 70°C. To this mixture, N-stearoyl glutamic acid-sodium glutamate, and sodium stearate were added and dissolved. Further added to this mixture was a premixed solution in which cetostearyl alcohol, hydrogenated palm oil, and mineral oil were mixed and evenly dissolved at 70°C. The mixture was emulsified by a homomixer and cooled down to a room temperature to give a milky solution. This milky solution left the skin moist and smooth.

As apparent from the above description, the invention provides a novel surfactant which is obtained by blending an N-C₈₋₂₄ acylamino acid and a certain base (i.e. an alkali salt of an amino acid). A detergent composition comprising this surfactant as a cleansing component ensures good lathering ability and foam quality, and does not cause stiff hair or taut skin after use. In addition, an emulsion composition comprising this surfactant as an emulsifying component ensures a desirable emulsion state.

### INDUSTRIAL APPLICABILITY

In particular, the surfactant of the invention is highly useful as a cleansing component for detergent compositions such as hair shampoos, body shampoos, and face washes, and is also highly useful as an emulsifying component for emulsion compositions such as cosmetic creams, milky lotions, and serums.

## Claims

1. A surfactant **characterised in** comprising a blend of an acidic or neutral N-₈₋₂₄ acylamino acid and a base , wherein
said base has an ion pair formed by an anion deriving from a carboxyl group of an amino acid and a cation deriving from alkali metals, alkaline earth metals, organic amines, basic amino acids, N-methyltaurine sodium, N-methyltaurine potassium, taurine sodium, taurine potassium, and
said acidic or neutral N-C₈₋₂₄ acylamino acid and said base are blended and neutralised to form an ion pair having a pH of 5 to 9, where
the amount of said base is 1.0 to 1.6 equivalents relative to 1 equivalent of N-C₈₋₂₄ acylamino acid and, in the case where the N-C₈₋₂₄ acylamino acid has two carboxl groups, is 1.3 to 2.3 equivalents relative to 2 equivalents of N-C₈₋₂₄ acylamino acid.

2. A surfactant according to claim 1, wherein an amino acid in the base is at least one member selected from the group consisting of acidic amino acids and neutral amino acids.

3. A surfactant according to claim 1, wherein an amino acid in the base is at least one member selected from α-amino acids.

4. A surfactant according to claim 1, wherein an amino acid in the base is at least one member selected from the group consisting of glycine, trimethylglycine, alanine, serine, proline, hydroxyproline, glutamine, glutamic acid, asparagine, aspartic acid, and glycylglycine.

5. A surfactant according to claim 1, wherein an amino acid in the base is at least one member selected from the group consisting of glycine, trimethylglycine, alanine, serine, glutamic acid, and glycylglycine.

6. A surfactant according to claim 1, wherein an cation in the base is at least one member selected from the group consisting of sodium, potassium, triethanolamine, and N-methyltaurine sodium.

7. A surfactant according to claim 1, wherein a C₈₋₂₄ acyl in the N-C₈₋₂₄ acylamino acid is a C₁₂₋₁₈ acyl.

8. A surfactant according to claim 1, wherein a C₈₋₂₄ acyl in the N-C₈₋₂₄ acylamino acid is at least one member selected from the group consisting of lauroyl, myristoyl, palmitoyl, stearoyl, isostearoyl, oleoyl, coconut oil acyl, palm oil acyl, palm kernel oil acyl, tallow acyl, and hydrogenated tallow acyl.

9. A surfactant according to claim 1, wherein an amino acid in the N-C₈₋₂₄ acylamino acid is at least one member selected from the group consisting of acidic amino acids and neutral amino acids.

10. A surfactant according to claim 1, wherein an amino acid in the N-C₈₋₂₄ acylamino acid is at least one member selected from the group consisting of glutamic acid, aspartic acid, sarcosine, alanine, glycine, beta -alanine, N-methyl- beta -alanine, and glutamine.

11. A detergent composition which comprises, as a cleansing component, a surfactant according to any of claims 1 to 10.

12. A detergent composition according to claim 11, wherein the surfactant content is 3 to 50% by weight.

13. An emulsion composition which comprises, as an emulsifying component, a surfactant according to any of claims 1 to 10.

14. An emulsion composition according to claim 13, wherein the surfactant content is 0.1 to 5% by weight.

## Patentansprüche

1. Ein Tensid **dadurch gekennzeichnet, dass** es eine Mischung von sauren oder neutralen N-C₈₋₂₄ Acylaminosäure und einer Base umfasst, wobei
besagte Base ein Ionenpaar aufweist, gebildet durch ein Anion, welches von einer Carboxylgruppe einer Aminosäure stammt von einem Kation abgeleitet von Alkalimetallen, Erdalkalimetallen, organischen Aminen, Basen und Aminosäuren, N-Methyltaurin, Kalium N-Methyltaurin, Natriumtaurin, Kaliumtaurin und
besagte saure oder neutrale N-C₈₋₂₄ Acylaminosäure und besagte Base gemischt und neutralisiert sind, um ein Ionenpaar mit einem pH von 5 bis 9 zu bilden, wobei
die Menge der besagten Base 1,0 bis 1,6 Äquivalente relativ zu einem Äquivalent auf N-C₈₋₂₄ Acylaminosäure und in dem Fall dass die N-C₈₋₂₄ Acylaminosäure zwei Carboxylgruppen hat 1,3 bis 2,3 Äquivalente relativ zu zwei Äquivalenten von N-C₈₋₂₄ Acylaminosäure ist.

2. Ein Tensid gemäß Anspruch 1, wobei die Aminosäure in der Base wenigstens ein Mitglied ausgewählt aus der Gruppe bestehend aus sauren Aminosäuren und neutralen Aminosäuren ist.

3. Ein Tensid gemäß Anspruch 1, wobei die Aminosäure in der Base wenigstens ein Mitglied ausgewählt aus α- Aminosäuren ist.

4. Ein Tensid gemäß Anspruch 1, wobei eine Aminosäure in der Base wenigstens ein Mitglied ausgewählt aus der Gruppe bestehend aus Glyzin, Trimethylglyzin, Alanin, Serin, Prolin, Hydroxyprolin, Glutamin, Glutaminsäure, Asparagine, Asparalinsäure und Glyzylglyzin ist.

5. Ein Tensid gemäß Anspruch 1, wobei eine Aminosäure in der Base wenigstens ein Mitglied ausgewählt aus der Gruppe bestehend aus Glyzin, Trimethylglyzin, Alanin, Serin, Glutamatsäure und Glyzylglyzin ist.

6. Ein Tensid gemäß Anspruch 1, wobei ein Kation in der Base wenigstens ein Mitglied ausgewählt aus der Gruppe bestehend aus Natrium, Kalium, Triethanolamin und N-Methyltaurinnatrium.

7. Ein Tensid gemäß Anspruch 1, wobei ein C₈₋₂₄ Acyl in der N-C₈₋₂₄ Acylaminosäure ein C₁₂₋₁₈ Acyl ist.

8. Ein Tensid gemäß Anspruch 1, wobei ein C₈₋₂₄ Acyl in der N-C₈₋₂₄ Aminosäure wenigstens ein Mitglied aus der Gruppe bestehend aus Laurolyl, Myristoyl, Palmitoyl, Stearoyl, Isostearoyl, Oleoyl, Acylkokosnussöl, Acylpalmöl, Acylpalmkernöl, Acyltalg, hydriertem Acyltalg ist.

9. Ein Tensid gemäß Anspruch 1, wobei eine Aminosäure in der N-C₈₋₂₄ Acylaminosäure wenigstens ein Mitglied ausgewählt aus der Gruppe bestehend aus sauren Aminosäuren und neutralen Aminosäuren ist.

10. Ein Tenisd gemäß Anspruch 1, wobei eine Aminosäure in der N-C₈₋₂₄ Acylaminosäure wenigstens ein Mitglied aus Gruppe besthend aus Glutamatsäure, Aspertatsäure, Sarkosin, Alanin, Glyzin, Beta-Alanin, N-Methyl-Beta-Alanin und Glutamin ist.

11. Eine Detergenzzusammensetzung umfassend eine Reinigungskomponente und eines Tensides gemäß eines der Ansprüche 1-10.

12. Eine Detergenzzusammensetzung gemäß Anspruch 11, wobei der Tensidgehalt zwischen 30 bis 50 Gewichts-% ist.

13. Eine Emulsionszusammensetzung umfassend eine emulierende Komponente und ein Tensid gemäß eines der Ansprüche 1-10.

14. Eine Emulsionszusammensetzung gemäß Anspruch 13, worin der Tensidgehalt zwischen 0,1 bis 5 Gewichts-% ist.

## Revendications

1. Surfactant **caractérisé en ce qu'**il comprend un mélange d'un acide acylamino N-C₈₋₂₄ neutre ou acide et une base, dans lequel
la dite base a une paire d'ions formée par un anion dérivant d'un groupe carboxylique d'un acide aminé et un cation dérivant de métaux alcalins, métaux alcalino-terreux, amines organiques, acides aminés basiques, sodium N-méthyltaurine, potassium-N-méthyltaurine, sodium taurine, potassium taurine, et
ledit acide acylamino N-C₈₋₂₄ neutre ou acide et ladite base sont mélangés et neutralisés pour former une paire d'ions ayant un pH de 5 à 9, où
la quantité de ladite base est de 1.0 à 1.6 en équivalent par rapport à un équivalent de l'acide acylamino N-C₈₋₂₄ et, dans le cas où l'acide acylamino N-C₈₋₂₄ a deux groupes carboxyliques, la quantité de ladite base est de 1.3 à 2.3 en équivalent par rapport à deux équivalents de l'acide acylamino N-C₈₋₂₄.

2. Surfactant selon la revendication 1, dans lequel un acide aminé dans la base est au moins un membre choisi dans le groupe constitué par les acides aminés acides et les acides aminés neutres.

3. Surfactant selon la revendication 1, dans lequel un acide aminé dans la base est au moins un membre choisi dans le groupe des acides α-aminés.

4. Surfactant selon la revendication 1, dans lequel un acide aminé dans la base est au moins un membre choisi dans le groupe constitué par : la glycine, la triméthyglycine, l'alanine, la sérine, la proline, l'hydroxyproline, la glutamine, l'acide glutamique, l'asparagine, l'acide aspartique, et la glycylglycine.

5. Surfactant selon la revendication 1, dans lequel un acide aminé dans la base est au moins un membre choisi dans le groupe constitué par la glycine, la tryméthylglycirle, l'alanine, la sérine, l'acide glutamique, et glycylglycine.

6. Surfactant selon la revendication 1, dans lequel un cation dans la base est au moins un membre sélectionné dans le groupe constitué par le sodium, le potassium, la triéthanolamine, et le sodium N-méthyltaurine.

7. Surfactant selon la revendication 1, dans lequel un groupe acyle C₈₋₂₄ dans l'acide acylamino N-C₈₋₂₄ est un acyle N-C₁₂₋₁₈.

8. Surfactant selon la revendication 1, dans lequel un groupe acyle C₈₋₂₄ dans l'acide acylamino N-C₈₋₂₄ est au moins membre choisi dans le groupe constitué par : lauroyl, myristoyl, palmitoyl, stéaroyl, isostéaroyl, oleoyl, acyle d'huile de noix de coco, acyle d'huile de palme, acyle d'huile de noyau de palme, acyle de suif, et acyle de suif hydrogéné.

9. Surfactant selon la revendication 1, dans lequel un acide aminé dans l'acide acylamino N-C₈₋₂₄ est au moins un membre choisi dans le groupe constitué par les acides aminés acides et les acides aminés neutre.

10. Surfactant selon la revendication 1, dans lequel un acide aminé dans l'acide acylamino N-C₈₋₂₄ est au moins un membre choisi dans le groupe constitué par : l'acide glutamique, l'acide aspartique, la sarcosine, l'alanine, la glycine, la béta-alanine, la N-méthyl-béta-alanine, et la glutamine.

11. Composition de détergent qui comprend, en tant qu'agent de nettoyage, un surfactant selon l'une quelconque des revendications 1 à 10.

12. Composition de détergent selon la revendication 11, dans lequel la quantité de surfactant est de 3 à 50% en poids.

13. Composition d'émulsion qui comprend, en tant que composant émulsifiant, un surfactant selon l'une quelconque des revendications 1 à 10.

14. Composition d'émulsion selon la revendication 13, dans laquelle la quantité de surfactant est de 0.1 à 5% en poids.
